Europäisches Patentamt

European Patent Office

Office européen des brevets

(11) Veröffentlichungsnummer: **0 262 732 B1**

# (12) EUROPÄISCHE PATENTSCHRIFT

(45) Veröffentlichungstag der Patentschrift: **15.07.92**

(21) Anmeldenummer: **87201824.7**

(22) Anmeldetag: **22.09.87**

(51) Int. Cl.⁵: **G06F  15/68**, G06F 15/62

(54) **Verfahren zum Erzeugen eines Röntgenschichtbildes eines Untersuchungsbereiches und Anordnung zur Durchführung des Verfahrens.**

(30) Priorität: **26.09.86 DE 3632833**

(43) Veröffentlichungstag der Anmeldung:
**06.04.88 Patentblatt  88/14**

(45) Bekanntmachung des Hinweises auf die
Patenterteilung:
**15.07.92 Patentblatt  92/29**

(84) Benannte Vertragsstaaten:
**DE FR GB**

(56) Entgegenhaltungen:
**EP-A- 0 106 402**

**MEDICAL PHYSICS, Band 12, Nr. 4,
Juli/August 1985, Seiten 431-436, Am. Assoc.
Phys. Med., New York, US; P. HAAKER et al.:
"A new digital tomosynthesis method with
less artifacts for angiography"**

(73) Patentinhaber: **Philips Patentverwaltung
GmbH
Wendenstrasse 35 Postfach 10 51 49
W-2000 Hamburg 1(DE)**

(84) Benannte Vertragsstaaten:
**DE**

(73) Patentinhaber: **N.V. Philips' Gloeilampenfabrieken
Groenewoudseweg 1
NL-5621 BA Eindhoven(NL)**

(84) Benannte Vertragsstaaten:
**FR GB**

(72) Erfinder: **Haaker, Paul Rudolf
Burgwedeltwiete 11
W-2000 Hamburg 61(DE)**
Erfinder: **Klotz, Erhard Paul Artur
Seekamp 110
W-2083 Halstenbek(DE)**
Erfinder: **Koppe, Reiner Heinrich, Dr.
Rugenbergener Weg 3
W-2000 Hamburg 61(DE)**
Erfinder: **Linde, Rolf Erich
Kamperrege 21
W-2081 Haseldorf(DE)**

(74) Vertreter: **Hartmann, Heinrich, Dipl.-Ing. et al
Philips Patentverwaltung GmbH Wendenstrasse 35 Postfach 10 51 49
W-2000 Hamburg 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 262 732 B1

**Beschreibung**

Die Erfindung betrifft ein Verfahren zum Erzeugen eines Röntgenschichtbildes eines Untersuchungsbereiches mit Hilfe von verschiedenen Perspektiven des Untersuchungsbereiches zugeordneten Einzelbildern, für deren Bildpunkte je ein Bildwert gespeichert ist, wobei jeder Bildwert eines Schichtbildpunktes durch jeweils einen der Bildwerte der diesem Schichbildpunkt geometrisch zugeordneten Bildpunkte der Einzelbilder gebildet wird, sowie eine Anordnung zur Durchführung des Verfahrens.

Ein solches Verfahren und eine solche Anordnung sind aus der DE-OS 32 37 572 bekannt. Die bekannte Anordnung besitzt vier Röntgenstrahler, die vier getrennte Einzelbilder eines zu durchstrahlenden Untersuchungsbereiches erzeugen. Die Einzelbilder sind dabei in eine Vielzahl von endliche Abmessungen aufweisende, vorzugsweise quadratische Bereiche unterteilt, die im folgenden als Bildpunkte bezeichnet werden, während mit Schichtbildpunkte ein entsprechender Bereich in dem aus den Einzelbildern hergestellten Schichtbild bezeichnet wird. Für jeden Bildpunkt eines Einzelbildes wird in einer Speicheranordnung der zugehörige Bildwert gespeichert, der ein Maß für die Absorption bzw. Transmission des Untersuchungsbereiches in dem jeweiligen Bildpunkt ist. Der Bildwert, der einem Schichtbildpunkt dabei zugeordnet wird, wird aus den Bildpunkten abgeleitet, die dem Schichtbildpunkt geometrisch zugeordnet sind. Diese Bildpunkte liegen bei der Röntgenaufnahme, aus der das betreffende Einzelbild gebildet wird, auf der Verbindungslinie zwischen dem Schichtbildpunkt mit den verschiedenen Röntgenstrahlern.

Bei dem bekannten Verfahren, das vorzugsweise zur Erzeugung von Schichtbildern der mit einem Kontrastmittel gefüllten Arterien der Herzkranzgefäße dient, wird einem Schichtbildpunkt von den ihm geometrisch zugeordneten Bildpunkten der Einzelbilder jeweils der Bildwert zugeordnet, der der geringsten Absorption entspricht. Dadurch soll erreicht werden, daß absorbierende Strukturen außerhalb der Schicht keine bzw. nur geringfügige Artefakte in dem Bild dieser Schicht hervorrufen.

Das bekannte Verfahren arbeitet zufriedenstellend, solange die Einzelbilder nicht durch ein Bildverarbeitungsverfahren entstanden sind, das "verdünnte" Abbildungen liefert, d.h. Bilder, die vor einem mehr oder weniger homogenen Hintergrund auf einer im Vergleich zur gesamten Bildfläche kleinen Fläche die für die (medizinische oder technische) Diagnose wichtigen Details mit von dem Hintergrund deutlich abweichenden Bildwerten enthalten.

Solche verdünnten Abbildungen können beispielsweise dann entstehen, wenn die Einzelbilder mittels eines Subtraktionsverfahrens erzeugt werden, bei dem für verschiedene Perspektiven jeweils von einer Röntgenaufnahme der mit einem Kontrastmittel gefüllten Herzkranzgefäße ("Füllungsaufnahme") jeweils eine Röntgenaufnahme des gleichen Bereichs, jedoch ohne Kontrastmittel ("Maskenbild"), subtrahiert wird; dieses lediglich den Hintergrund darstellende Maskenbild kann auch synthetisch aus einer Füllungsaufnahme erzeugt werden, wie in der DE-OS 35 14 683 angegeben ist. Das sich dabei ergebende Einzelbild läßt praktisch kaum noch Skelett- und Weichteilstrukturen erkennen, sondern zeigt lediglich die mit dem Kontrastmittel gefüllten Gefäße, wobei die zugehörigen Bildwerte deutlich von den Bildwerten des Untergrundes abweichen.

Eine verdünnte Abbildung ergibt sich auch, wenn Röntgenaufnahmen einem Mustererkennungsverfahren unterzogen werden. Ein solches Mustererkennungsverfahren kann beispielsweise dazu dienen, um in Röntgenaufnahmen von Gußteilen die Gaseinschlüsse (Lunker) hervorzuheben, die sich im unverarbeiteten Röntgenbild praktisch kaum von ihrer Umgebung abheben. Dies kann beispielsweise dadurch erfolgen, daß jeder Bildwert mit einem Schwellenwert verglichen und - je nachdem, ob er größer oder kleiner ist als der Schwellenwert - durch einen ersten (Merkmal erkannt) oder zweiten (Merkmal nicht erkannt) Wert ersetzt wird.

Beide Verfahren haben gemeinsam, daß in den Einzelbildern, die für die Diagnose wichtigen Details (Gefäße bzw. Lunker) nur einen vergleichsweise kleinen Anteil der Gesamtfläche eines Einzelbildes bedecken, daß die Bildwerte in den betreffenden Bildpunkten sich jedoch wesentlich von denen des Unter- bzw. Hintergrundes unterscheiden. Während jedoch im ersten Fall die Absorption der für die Diagnose wichtigen Details größer ist als die der Umgebung, ist die Absorption im zweiten Fall kleiner (bzw. die Transmission ist größer) als in der Umgebung. Wendet man das bekannte Verfahren auf derartige Einzelbilder an, dann können in dem Schichtbild Artefakte entstehen.

Aufgabe der vorliegenden Erfindung ist es, ein Verfahren der eingangs genannten Art so auszugestalten, daß die Artefakte im Falle von derartigen "verdünnten" Einzelbildern stärker unterdrückt werden.

Diese Aufgabe wird dadurch gelöst, daß jeweils derjenige Bildwert als Bildwert des Schichtbildpunktes eingesetzt wird, der der zweitniedrigsten Absorption entspricht.

Die Erfindung basiert auf folgenden Überlegungen:
Bei der Erzeugung der Einzelbilder können Fehler entstehen derart, daß bei einem für die Diagnose wichtigen Detail ein Bildwert zugeordnet wird, der dem Hintergrund entspricht. Wenn beispielsweise bei dem Subtraktionsverfahren nach der DE-OS 35 14 683 das aus einer Anzahl von Füllungsaufnahmen erzeugte und von dieser zu subtrahierende Maskenbild einem der Bildpunkte eines Einzelbildes fälschlicherweise einen Bildwert zuordnet, der dem für die Diagnose wichtigen Detail (Gefäß) entspricht, dann macht sich dieser Fehler dadurch bemerkbar, daß in dem durch Subtraktion gebildeten Einzelbild dieser Gefäßpunkt nicht erscheint, so daß der Bildwert für diesen Bildpunkt einer wesentlich kleineren Absorption entspricht, als wenn der Verarbeitungsfehler nicht auftreten würde. Bei der Erzeugung des Schichtbildes aus den Einzelbildern würde bei dem bekannten Verfahren für den Schichtbildpunkt, dem der genannte Gefäß-Bildpunkt mit dem fehlerhaften Bildwert geometrisch zugeordnet ist, eben dieser fehlerhafte Bildwert erzeugt, weil dieser der niedrigsten Absorption entspricht, d.h. daß immer dann, wenn bei der Erzeugung irgendeines Einzelbildes durch Subtraktion eines Maskenbildes von einem Füllungsbild ein Gefäß-Bildpunkt als Hintergrund-Bildpunkt erscheint, dieser Fehler sich in das aus diesem und weiteren Einzelbildern erzeugte Schichtbild überträgt.

Die Erfindung nutzt die Tatsache aus, daß diese Bildfehler in den Einzelbildern nicht korreliert sind, d.h. daß in der Regel von den Bildwerten der einem Schichtbildpunkt geometrisch zugeordneten Bildpunkte der Einzelbilder allenfalls einer den geschilderten Fehler aufweist. Da nun gemäß der Erfindung der Bildwert eingesetzt wird, der der zweitkleinsten Absorption entspricht, spielt der geschilderte Fehler für die Bestimmung des Bildwertes eines Schichtbildpunktes keine Rolle mehr, so daß er sich in der Regel nicht in das Schichtbild überträgt.

Auch die Fehler in den Einzelbildern, die dadurch entstehen, daß bei der Subtraktion ein (schwächer absorbierender) Hintergrund-Bildpunkt als (stärker absorbierender) Gefäß-Bildpunkt erscheint, werden unterdrückt, weil der Bildwert dieses Bildpunktes der größten Absorption entspricht und daher nicht zur Bildung des Bildwertes für einen Schichtbildpunkt herangezogen wird. Auch werden die durch absorbierende Strukturen außerhalb der Schicht bedingten Artefakte in dem Schichtbild weitgehend unterdrückt.

Eine ähnliche Unterdrückung von Artefakten liefert die Erfindung auch dann, wenn die Einzelbilder, mit deren Hilfe ein Schichtbild erzeugt werden soll, Produkt eines Mustererkennungsverfahrens sind.

Eine Anordnung zur Durchführung des erfindungsgemäßen Verfahrens ist gekennzeichnet durch mehrere Strahlenquellen zur Erzeugung von Röntgenaufnahmen des Untersuchungsbereichs unter verschiedenen Perspektiven, Mittel zum Verarbeiten der Röntgenaufnahmen zu Einzelbildern, eine Speicheranordnung zum Speichern der Bildwerte der Einzelbilder und durch Mittel zum Bestimmen desjenigen Bildwertes, der einem Schichtbildpunkt geometrisch zugeordneten Bildpunkte, der der zweitniedrigsten Absorption bzw. Transmission entspricht.

Die Erfindung wird nachstehend anhand der Zeichnung näher erläutert. Es zeigen:

Fig. 1 eine Anordnung zur Durchführung des Verfahrens in schematischer Darstellung und

Fig. 2 ein Flußdiagramm zur Bestimmung des Bildwertes eines Schichtbildpunktes.

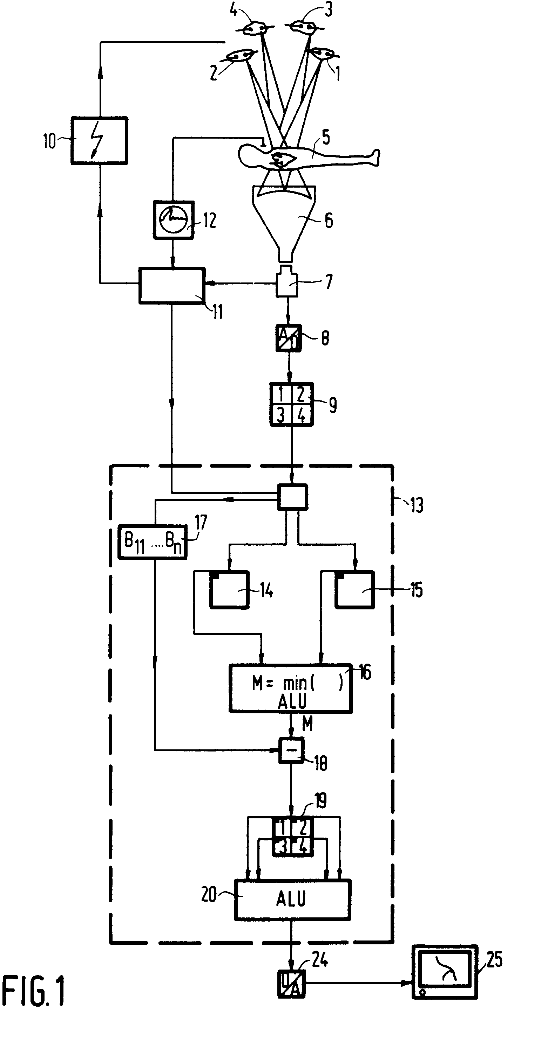

Fig. 1 zeigt vier auf den Eckpunkten eines Quadrates angeordnete Röntgenstrahler 1, 2, 3 und 4, deren Strahlenbündel auf einen Untersuchungsbereich ausgerichtet sind, in dem sich die mit einem Kontrastmittel gefüllten Herzkranzgefäße eines Patienten 5 befinden. Ein Röntgenbildverstärker 6 wandelt die von den Röntgenröhren 1...4 erzeugten Röntgenschattenbilder in sichtbare Bilder um. Diese werden mittels einer Fernsehkamera 7 in elektrische Signale umgesetzt, die von einem Analog-Digital-Wandler 8 digitalisiert und in einem Speicher 9 gespeichert werden. Die Röntgenröhren 1...4 werden jeweils gleichzeitig von einem Hochspannungsgenerator 10 eingeschaltet, der von einem Triggermodul 11 gesteuert wird, der seine Triggersignale aus einem Elektrokardiogramm ableitet, die von einer an den Patienten 5 angeschlossenen EKG-Einrichtung 12 geliefert werden. Auf diese Weise lassen sich die Röntgenstrahler 1...4 in definierten Phasen der Herzaktion einschalten.

Das Einschalten erfolgt n mal in unterschiedlichen Phasen der Herzaktion, so daß für jeden Röntgenstrahler 1...4 eine Folge von n Röntgenaufnahmen B11...B1n,...,B41...B4n in dem Speicher 9 gespeichert ist, die den Untersuchungsbereich in unterschiedlichen Phasen der Herzaktion darstellen. Aus den gespeicherten Bildern werden in einer Recheneinrichtung 13 Bilder von Schichten mit einer vorgebbaren Lage und für verschiedene Phasen der Herzaktion berechnet. Diese Bilder werden über einen Digital-Analog-Wandler 14 einer geeigneten Bildwiedergabeeinrichtung 15, z.B. einem Monitor, zugeführt. Die Berechnung der Schichtbilder erfolgt in zwei Phasen. In der ersten Phase werden aus den gespeicherten Röntgenaufnahmen Einzelbilder errechnet. In der zweiten Phase wird aus vier Einzelbildern, die die Herzkranzgefäße jeweils in der gleichen Phase der Herzaktion aber aus unterschiedlichen Perspektiven zeigen, wird mindestens ein Schichtbild errechnet. Die Berechnung der Einzelbilder erfolgt - für jeden Röntgenstrahler getrennt - auf folgende Weise:

Aus der Folge der Röntgenaufnahmen für einen Röntgenstrahler, z.B. den Röntgenaufnahmen B11..B1n des Röntgenstrahlers 1, werden einige ausgewählt, die die Herzkranzgefäße in deutlich unterschiedlichen Bewegungsphasen zeigen. Von diesen werden zunächst zwei in die Speicher 14 und 15 geladen. Eine arithmetisch logische Einheit 16 vergleicht die beiden Bilder Bildpunkt für Bildpunkt und speichert jeweils denjenigen Bildwert, der der geringeren Absorption entspricht. Das so entstandene Bild wird in einen der Speicher 14 und 15 geladen und seinerseits mit dem nächsten der ausgewählten Bilder der Folge verglichen. Am Ende ist ein Bild entstanden, dessen Bildwerte den Minima der Absorption in den ausgewählten Bildern entsprechen.

Da in den Röntgenaufnahmen zwischen den Herzkranzgefäßen und dem Hintergrund ein merkbarer Kontrast vorhanden ist und die Herzkranzgefäße sich vor dem als fest angenommenen Hintergrund bewegen und diesen in einzelnen Phasen, d.h. in einzelnen der ausgewählten Bilder, freigeben, ergibt sich ein Minimum der Absorption für jeden Bildpunkt jeweils dann, wenn dieser Bildpunkt in den ausgewählten Röntgenaufnahmen wenigstens einmal nicht durch ein bewegtes Herzkranzgefäß verdeckt war. Der Bildwert für diesen Bildpunkt entspricht somit dem Hintergrund und das auf diese Weise erzeugte Bild zeigt daher praktisch nur den Hintergrund, aber keine Herzkranzgefäße mehr. Dieses Bild kann als Maskenbild M benutzt werden, das von den Röntgenaufnahmen B11...B1n der Folge, die in einen Speicher 17 geladen worden sind, in der Subtraktionsschaltung 18 subtrahiert werden. Die Folge der Röntgenaufnahmen wird dadurch in eine Folge von Einzelbildern umgesetzt, die im wesentlichen nur die Gefäße zeigen, während der Hintergrund unterdrückt ist, weil die Röntgenaufnahmen sowohl die Gefäße als auch den Hintergrund und das davon subtrahierte Maskenbild nur den Hintergrund enthalten. Dieses in der deutschen Patentanmeldung P 35 14 683 näher erläuterte Verfahren wird für die Folgen B21...B2n,...,B41...B4n der übrigen Röntgenstrahler 2...4 wiederholt, so daß am Ende des Verfahrens vier Folgen von Einzelbildern - für jede Röntgenröhre eine - zur Verfügung stehen. Zur Erzeugung eines Schichtbildes für eine bestimmte Herzphase werden die vier zu dieser Herzphase gehörenden Einzelbilder aus den vier Folgen in eine Speicheranordnung 19 geladen und es werden - wie in der DE-OS 32 37 572 näher beschrieben - diejenigen Bildpunkte ermittelt, die dem gleichen Schichtbildpunkt zugeordnet sind.

Wenn die ausgewählten Einzelbilder keine Fehler enthalten würden, würde der Bildwert, der der geringsten Absorption entspricht, zumindest annähernd dem Bildwert des Schichtbildpunktes entsprechen, wie im einzelen in der DE-OS 32 37 572 ausgeführt. Es kann jedoch vorkommen, daß in allen zur Erzeugung des Maskenbildes ausgewählten Röntgenaufnahmen ein oder mehrere Bildpunkte durch Gefäße verdeckt sind. Diese Bildpunkte gehen daher in das Maskenbild ein. Die Bildwerte für diese Bildpunkte haben praktisch die gleiche Größe wie die Bildwerte in den unverarbeiteten Röntgenaufnahme der Folge, so daß sie sich nach der Subtraktion kompensieren und das durch die Subtraktion entstandene Einzelbild für diesen Bildpunkt einen Bildwert aufweist, der einen minimalen Absorption entspricht. Würde als Bildwert eines Schichtbildpunktes von den geometrisch zugeordneten Bildpunkten jeweils der Bildwert herangezogen, der der kleinsten Absorption entspricht, dann würde der fehlerhafte Bildwert dieses Bildpunktes den Bildwert des räumlich zugeordneten Schichtbildpunktes bilden, d.h. das Schichtbild würde in diesem Bildpunkt kein Gefäß zeigen - und zwar auch dann, wenn die Bildwerte aus den anderen Einzelbildern für diesen Bildpunkt einem Gefäß entsprechen.

Erfahrungsgemäß treten die geschilderten Fehler aber nicht räumlich korreliert auf, d.h. die anderen Bilder werden in den Bildpunkten, die dem gleichen Schichtbildpunkt zugeordnet sind, in der Regel nicht den gleichen Fehler aufweisen. Wenn daher als Bildwert für den Schichtbildpunkt aus den vier Einzelbildern der Bildwert der geometrisch zugeordneten Bildpunkte gewählt wird, der der zweitkleinsten Absorption entspricht, dann wird der betreffende Schichtbildpunkt ein Gefäß zeigen - wenn sich das Gefäß in der abgebildeten Schicht befindet, d.h. in diesem Fall überträgt sich der in einem Einzelbild enthaltene Fehler nicht in das daraus hergestellte Schichtbild.

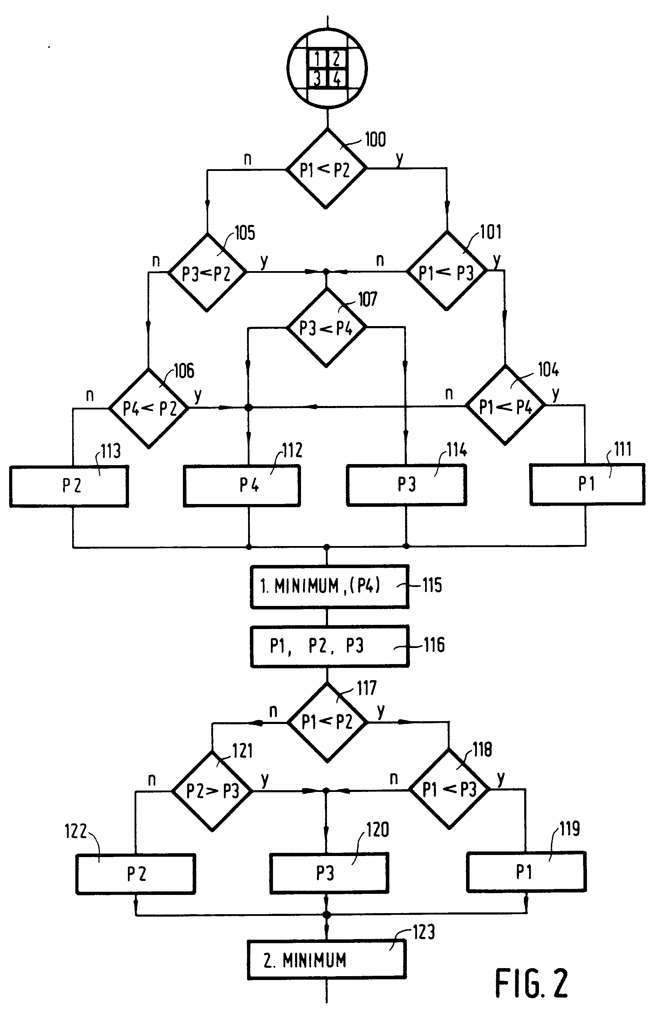

Das Verfahren, mit dem der Bildwert bestimmt werden kann, der der zweitkleinsten Absorption entspricht, wird anhand des in Fig. 2 dargestellten Flußdiagramms näher erläutert. Dabei wird davon ausgegangen, daß die vier Bildwerte P1, P2, P3, P4 der Bildpunkte aus den Einzelbildern, die einem Schichtbildpunkt geometrisch zugeordnet sind, gespeichert vorliegen. Zunächst werden P1 und P2 verglichen (Block 100). Ist P1 kleiner als P2, dann erfolgt eine Verzweigung zum Block 101, in dem P1 mit P3 verglichen wird. Ist P1 wiederum kleiner als P3, erfolgt eine Verzweigung zum Block 104, in dem P1 mit P4 verglichen wird. Ist P1 kleiner als P4, dann ist P1 der kleinste Wert (Block 111); anderenfalls ist P4 der kleinste Wert (Block 112).

Wenn P1 nicht kleiner ist als P2, erfolgt eine Verzweigung zum Block 105, in dem P2 und P3 miteinander verglichen werden. Ist P3 größer als P2, erfolgt eine Verzweigung zum Block 106, in dem P4 und P2 verglichen werden. Ist P4 wiederum größer als P2, dann ist P2 der kleinste Bildwert (Block 113); anderenfalls ist es wiederum P4.

Wenn der Vergleich im Block 105 ergibt, daß P3 kleiner ist als P2 oder wenn der Vergleich in Block 101 ergibt, daß P1 nicht kleiner ist als P3, erfolgt eine Verzweigung zum Block 107, in dem P3 und P4 miteinander verglichen werden. Je nach Ausgang dieses Vergleichs ist entweder P3 (Block 114) oder P4 der kleinste Bildwert. Wenn somit einer der Blöcke 111...114 erreicht worden ist, steht fest, welcher Bildwert der kleinste ist bzw. der kleinsten Absorption entspricht (Block 115). Wenn dies z.B. der Bildwert P4 ist, verbleiben lediglich noch die drei Bildwerte P1, P2, P3 (Block 116), unter denen der kleinste bestimmt werden muß. Dies ist dann der zweitkleinste unter den vier Bildwerten P1...P4. Die Bestimmung dieses Wertes geschieht auf folgende Weise:

Im Block 117 wird P1 mit P2 verglichen. Wenn P1 kleiner ist als P2 erfolgt eine Verzweigung zum Block 118, in dem P1 und P3 miteinander verglichen werden. Ist P1 wiederum kleiner als P3, dann wird P1 als kleinster Bildwert gespeichert (Block 119) und anderenfalls P3 (Block 120) Ergibt der Vergleich in Block 117 hingegen, daß P1 nicht kleiner ist als P2, dann erfolgt eine Verzweigung zum Block 121, in dem P2 mit P3 verglichen wird. Ist P2 nicht größer als P3, dann wird P2 als kleinster der drei Bildwerte gespeichert (Block 122) und anderenfalls P3. Nach der Durchführung dieser beiden weiteren Vergleiche (117 und 118 oder 117 und 121) steht also fest, welches der kleinste der drei verbliebenen Bildwerte und der zweitkleinste von insgesamt vier Bildwerten P1...P4 ist (Block 123). Dieser Wert wird als Bildwert des Schichtbildpunktes eingesetzt. Dies wird für alle Schichtbildpunkte wiederholt, bis aus den vier Einzelbildern ein Schichtbild erzeugt ist.

Es können danach mit den gleichen Einzelbildern Schichtbilder erzeugt werden, die eine tiefer oder höher im Objekt liegende Schicht darstellen. Es können aber auch aus vier Einzelbildern, die die Herzkranzgefäße in einer anderen Phase der Herzaktion zeigen, ein oder mehrere Schichtbilder gewonnen werden.

Obwohl vorstehend die Erfindung anhand eines Ausführungsbeispiels erläutert wurde, bei dem das Maskenbild aus Röntgen-Füllungsaufnahmen abgeleitet wurde, ist die Erfindung auch bei Subtraktionsverfahren anwendbar, bei denen das Maskenbild auf andere Weise erzeugt wird, z.B. so, wie in der Zeitschrift "Radiology" Vol. 151, Nr. 2, Seiten 517 bis 520, beschrieben.

Auch ist die Erfindung nicht nur bei solchen Verfahren anwendbar, bei denen die Einzelbilder durch ein Subtraktionsverfahren aus den Röntgenaufnahmen abgeleitet werden, sondern auch bei Verfahren, bei denen die Einzelbilder durch ein Mustererkennungsverfahren aus den Röntgenaufnahmen abgeleitet werden. Damit können Fehler in Werkstücken, beispielsweise Lunker in Gußstücken, erkannt werden, wobei die beim Mustererkennungsverfahren eingeführten Fehler in einzelnen Bildpunkten der Einzelbilder durch die Schichtbilderzeugung weitgehend eliminiert werden.

**Patentansprüche**

1. Verfahren zum Erzeugen eines Röntgenschichtbildes eines Untersuchungsbereiches mit Hilfe von verschiedenen Perspektiven des Untersuchungsbereiches zugeordneten Einzelbildern, für deren Bildpunkte je ein Bildwert gespeichert ist, wobei jeder Bildwert eines Schichtbildpunktes durch jeweils einen der Bildwerte der diesem Schichtbildpunkt geometrisch zugeordneten Bildpunkte der Einzelbilder gebildet wird,
dadurch gekennzeichnet, daß jeweils derjenige Bildwert als Bildwert des Schichtbildpunktes eingesetzt wird, der der zweitniedrigsten Absorption entspricht.

2. Verfahren nach Anspruch 1 für die medizinische Röntgendiagnostik,
dadurch gekennzeichnet, daß die Einzelbilder durch Subtraktion jeweils eines Maskenbildes (B11...B4n) von einer Füllungsaufnahme gebildet werden.

3. Verfahren nach Anspruch 1,
dadurch gekennzeichnet, daß die Einzelbilder aus nach einem Mustererkennungsverfahren verarbeiteten, den Untersuchungsbereich unter verschiedenen Perspektiven darstellenden Röntgenaufnahmen erzeugt werden.

4. Anordnung zum Durchführen des Verfahrens nach einem der vorhergehenden Ansprüche mit
mehreren Strahlenquellen (1...4) zur Erzeugung von Röntgenaufnahmen (B11...B4n) des Untersuchungsbereichs unter verschiedenen Perspektiven, Mitteln (14...18) zum Verarbeiten der Röntgenaufnahmen zu Einzelbildern, einer Speicheranordnung (19) zum Speichern der Bildwerte der Einzelbilder und Mitteln (20) zum Bestimmen desjenigen Bildwertes der einem Schichtbildpunkt geometrisch zugeordneten Bildpunkte,
dadurch gekennzeichnet, daß die Mittel zum Bestimmen der Bildwerte denjenigen Bildwert der einem Schichtbildpunkt geometrisch zugeordneten Bildpunkte bestimmt, der der zweitniedrigsten Absorption entspricht.

**Claims**

1. A method of generating an X-ray layer image of an examination zone by means of single images which are associated with different perspectives of the examination zone and for the image points of which there is stored a respective image value, each image value of a layer image point being formed by one of the image values of the image points of the single images geometrically associated with the respective layer image point, characterized in that each time the image value which corresponds to the next tot the lowest absorption is used as the image value for the layer image point.

2. A method as claimed in Claim 1, for medical X-ray diagnostics, characterized in that the single images are formed by subtraction of a mask image (B11...B4n) from a filled exposure.

3. A method as claimed in Claim 1, characterized in that the single images are derived from X-ray images which are processed in accordance with a pattern recognition method and which depict the examination zone from different perspectives.

4. A device for performing the method claimed in any one of the preceding Claims, comprising a plurality of radiation sources (1...4) for forming X-ray images (B11...B4n) of the examination zone from different perspectives, means (14...18) for processing the X-ray images so as to form single images, a storage device (19) for storing the image values of the single images, and means (20) for determining that image value of the image points geometrically associated with a layer image point, characterized in that the means for determining the image values determine that image value of the image points geometrically associated with a layer image point which corresponds to the next to the lowest absorption.

**Revendications**

1. Procédé pour la production d'une image tomographique de rayons X d'un domaine d'examen à l'aide d'images individuelles associées à diverses perspectives du domaine d'examen, pour les points d'image desquels une valeur d'image est chaque fois stockée, chaque valeur d'image d'un point d'image tomographique étant chaque fois formée par une des valeurs d'image des points d'image des images individuelles associés géométriquement à ce point d'image tomographique, caractérisé en ce que l'on utilise chaque fois comme valeur d'image du point d'image tomographique, la valeur d'image qui correspond à la deuxième plus faible absorption.

2. Procédé suivant la revendication 1 pour le radiodiagnostic médical, caractérisé en ce que les images individuelles sont formées par soustraction de chaque fois une image de masque (B11 ... B4n) d'une image avec remplissage.

3. Procédé suivant la revendication 1, caractérisé en ce que les images individuelles sont produites à partir d'images radiographiques traitées selon un procédé de reconnaissance de structure, qui représentent le domaine d'examen dans diverses perspectives.

4. Montage pour l'exécution du procédé suivant l'une quelconque des revendications précédentes, comportant plusieurs sources de rayonnement (1 ... 4) servant à produire des images radiographiques (B11 ... B4n) du domaine d'examen dans diverses perspectives, des moyens (14 ... 18) pour transformer les images radiographiques en images individuelles, un dispositif de stockage (19) pour stocker les valeurs d'image des images individuelles et des moyens (20) pour déterminer la valeur d'image des points d'image associés géométriquement à un point d'image tomographique, caractérisé en ce que les moyens prévus pour déterminer les valeurs d'image déterminent la valeur d'image des points d'image associés géométriquement à un point d'image tomographique qui correspond à la deuxième plus petite absorption.

FIG. 1

FIG. 2